# EUROPEAN PATENT SPECIFICATION

(11) **EP 1 419 792 B1**
(45) Date of publication and mention of the grant of the patent: **02.07.2008**
(21) Application number: 02755730.5
(22) Date of filing: 31.07.2002
(51) Int. Cl.: A61L 27/20, A61L 27/44, A61L 27/50, C08G 65/322, C12M 3/00, C08B 37/00, A61P 19/02, A61L 27/24

(54) **GLYCOSAMINOGLYCAN-POLYCATION COMPLEX CROSSLINKED BY POLYFUNCTIONAL CROSSLINKING AGENT AND PROCESS FOR PRODUCING THE SAME**
DURCH EIN POLYFUNKTIONALES VERNETZUNGSMITTEL VERNETZTER GLYCOSAMINOGLYCAN-POLYKATION-KOMPLEX UND VERFAHREN ZU SEINER HERSTELLUNG
COMPLEXE POLYCATION-GLYCOSAMINOGLYCANE RETICULE PAR UN AGENT DE RETICULATION POLYFONCTIONNEL ET PROCEDE DE PRODUCTION ASSOCIE

(30) Priority: 21.08.2001 JP 2001250856
(43) Date of publication of application: 19.05.2004
(73) Proprietor: Japan Science and Technology Agency, Kawaguchi-shi, Saitama 332-0012 (JP); National Institute for Materials Science, Tsukuba-shi, Ibaraki 305-0047 (JP); NITTA GELATIN INC., Osaka-shi Osaka 556-0022 (JP)
(72) Inventor: TANAKA, Junzo, Tsukuba-shi, Ibaraki 305-0032 (JP); TAGUCHI, Tetsushi, Tsukuba-shi, Ibaraki 305-0032 (JP); MIYAZAKI, Tadasuke, Hachioji-shi, Tokyo 192-0904 (JP); SAKURA, Yoshiyuki, Kanazawa-ku, Kanagawa 236-0014 (JP); OHTSUKA, Tatsuro, Amagasaki-shi, Hyogo 660-0052 (JP); MANDAI, Yoshinobu, Yao-shi, Osaka 581-0042 (JP)
(74) Representative: Wagner, Karl H.
(86) International application number: PCT/JP2002/007824
(87) International publication number: WO 2003/028781

(56) References cited:
- EP-A1- 0 656 215
- EP-A1- 0 680 990
- EP-A1- 0 732 109
- WO-A-95/26761
- WO-A-97/22371
- WO-A1-97/22372

## Description

### TECHNICAL FIELD

The present invention relates to a matrix material for use in tissue regeneration such as cartilage repair, and more particularly to a glycosaminoglycan-polycation complex formed by a crosslinking reaction in the presence of calls using a polyfunctional crosslinking agent, and a preparation method thereof.

### BACKGROUND ART

It is known that, once damaged, articular cartilage will have serious difficulties in its tissue regeneration. According to worldwide statistics, it is reported that the number of patients of osteoarthritis caused by aging and sports injuries is no fewer than about ten million (about 1.2 million in Japan). In this context, it is strongly desired to develop a material for cartilage regeneration.

Heretofore, a complex of glycosaminoglycan (hyaluronic acid: HyA or chondroitin sulfate: ChS) and polycation (collagen: Col), which are primary components of cartilage tissue, has been prepared through a method of chemically crosslinking polyion complexes.

Such a crosslinked product is disclosed, for example, in Japanese Patent Laid-Open Publication Nos. 08-34747, 08-53548, 08-502082, 09-249751, 10-501706, 11-509256, 2000-501975 and 2000-502380.

In methods disclosed in the above Japanese Patent Laid-Open Publication Nos. 09-249751, 08-34747 and 2000-501975, a crosslinking reaction is conducted in alcohol or water, and a resulting injectable crosslinked biomaterial composition would have an adverse affect on cells and tissues (cell death). Thus, there is the need for an improved crosslinking method capable of avoiding this problem.

The above Japanese Patent Laid-Open Publication No. 10-501706 discloses that cells can be enclosed within a gel formed by crosslinking. However, any cell (membrane) will be destroyed due to the difference in osmotic pressure. Further, it is practically impossible to achieve the coexistence between collagen and glycosaminoglycan in water, and consequently the enclosing of cells is unrealizable.

While the above Japanese Patent Laid-Open Publication No. 09-249751 includes a description that collagen and glycosaminoglycan can be crosslinked together by a polyfunctional crosslinking agent, any adequate crosslinked product cannot be practically obtained because a polyion complex (inhomogeneous precipitate) will be undesirably formed due to plus charges of the collagen and minus charges of the glycosaminoglycan. Thus, it is required to develop an improved crosslinking method involving no formation of the undesirable polyion complex

### DISCLOSURE OF INVENTION

The present invention provides a novel crosslinked complex of polycation and glycosaminoglycan, which are primary components of the extracellular matrix of an articular cartilage.

Specifically, according to the present invention, there is provided a cartilage tissue regeneration matrix comprising of a biologically active glycosaminoglycan-polycation complex, which is formed by a homogeneous crosslinking reaction in the presence of calls using a polyfunctional crosslinking agent under physiological conditions. The crosslinking agent consists of polyethyleneglycol having two or more electrophilic leaving groups at the carboxyl terminal thereof.

The present invention also provides a method of preparing a cartilage tissue regeneration matrix comprising of a biologically active glycosaminoglycan-polycation complex comprising homogeneously crosslinking glycosaminoglycan and polycation with a polyfunctional crosslinking agent under physiological conditions to synthesize the glycosaminoglycan-polycation complex. In this method, the crosslinking agent consists of polyethyleneglycol having two or more electrophilic leaving groups at the carboxyl terminal thereof, and the concentration of the crosslinking agent is in the range of 0.3 to 3 mM.

In the above method, the crosslinking reaction between the glycosaminoglycan and polycation may be homogeneously conducted in the presence of cells mixed therewith in advance.

According to the preparation method of the present invention, the synthetic reaction can be conducted under physiological conditions, or under the conditions of pH 7.0 to 8.0, 37°C and 0.1 to 0.2 M NaCl. Thus, the crosslinking reaction between the glycosaminoglycan and polycation can be conducted in the presence of cells mixed therewith in advance, preferably, at a cell concentration in the range of 1 × 10⁸ cells/mL to 1 × 10⁴ cells/mL. The crosslinking reaction in a solution adjusted at a physiological pH and a physiological salt concentration allows the cells to be enclosed in a resulting formed gel in their living state. Additionally, an ion contained in vivo, such as calcium ion, magnesium ion or potassium ion may be added to the solution according to need. When cells are mixed, the concentration of collagen or glycosaminoglycan (GAG) is preferably set in the range of 0.5 to 5 wt%.

This means that a collagen/glycosaminoglycan/cell body (tissue-like structure) having the shape of a treated part (e.g. the shape of a lost cartilage) can be prepared. The crosslinking agent for use in the method of the present invention has low cytotoxicity, and thus an obtained complex can be used as a tissue generation matrix to be injected into bone, cartilage or nucleus pulposus by a syringe.

The crosslinked complex of the present invention has excellent properties as a tissue regeneration material for cartilage, nucleus pulposus, liver or blood vessel, because a crosslinking density can be easily controlled to allow the crosslinked complex to have a water content of 90 to 99 weight%, and the crosslinked complex can be decomposed by collagenase. In particular, when the weight ratio of glycosaminoglycan to polycation is in the range of 50 : 50 to 1 : 99, the crosslinked complex exhibits properties fairly similar to those of cartilage.

### BRIEF DESCRIPTION OF DRAWINGS

FIG. 1 is a graph showing a swelling degree of type-II collagen crosslinked at various pH values with a tetrafunctional crosslinking agent consisting of polyethyleneglycol having a succinimidyl group at the carboxyl terminal thereof.

FIG. 2 is a graph showing the transmittance of each product formed under the condition that a salt is added at various concentrations into a phosphoric acid buffer solution of pH 7.4.

FIG. 3 is a graph showing a swelling degree of each of collagen-glycosaminoglycan complex matrixes obtained in Inventive Examples 1 to 5 and Comparative Examples 1 and 2.

FIG. 4 is a photograph showing cartilage cells enclosed in a collagen-glycosaminoglycan complex matrix obtained in Inventive Example 6.

### BEST MODE FOR CARRYING OUT THE INVENTION

The electrophilic leaving group of the crosslinking agent for use in the synthetic method of the present invention may include a succinimidyl group, a sulfosuccinimidyl group and derivatives thereof. The crosslinking agent includes a pentaerythritol-based tetrafunctional crosslinking agent, an ethyleneglycol-based bifunctional crosslinking agent, a glycerin-based trifunctional crosslinking agent, and a hexaethyleneglycol-based octafunctional crosslinking agent. The polyethyleneglycol may have, but is not limited to, a molecular weight of 1000 or more.

The polycation to be combined with the glycosaminoglycan (GAG) (which is not limited to a specific type) includes: collagen (any one of several ten types) and derivatives thereof; gelatin (which is not limited to a specific molecular weight) as denatured collagen; polylysine (which is not limited to a specific molecular weight); and polymer molecule having an amino group such as chitosan (which is not limited to a specific deacetylation degree and molecular weight). Preferably, the collagen is preferably atelocollagen (i.e. collagen without telopeptide at its terminal).

The crosslinking agent will be described in more detail in conjunction with a tetrafunctional crosslinking agent consisting of polyethyleneglycol having a succinimidyl group at the carboxyl terminal thereof (Pentaerythritol polyethyleneglycol ether tetra succinimidyl glutarate) shown in the following chemical formula.

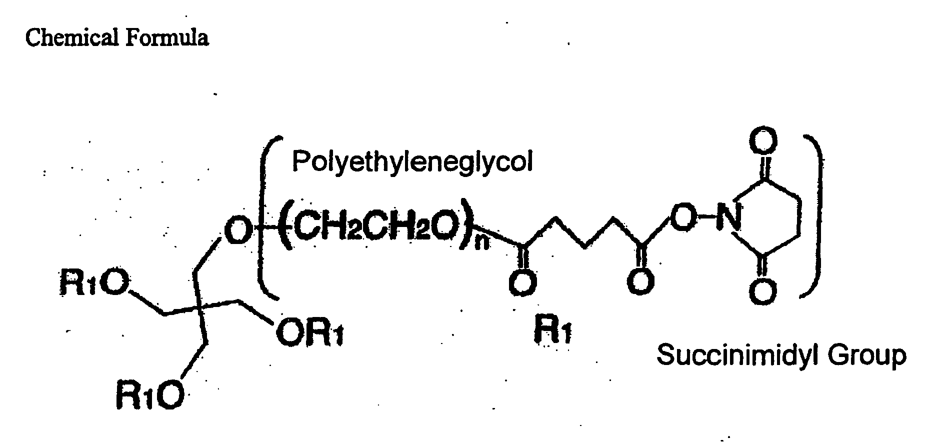

Ester hydrolysis is accelerated at a pH value of 7 or more, and thereby the succinimidyl group can induce a crosslinking reaction under physiological conditions.

In the succinimidylated carboxyl group at the carboxyl terminal of polyethyleneglycol, the succinimidyl group will be separated under a pH atmosphere of 7 or more. The carboxyl group after the succinimidyl group is separated therefrom reacts with the hydroxyl group or amino group of the GAG to crosslink between the respective molecules of the collagen and the GAG, and the molecules in each of the collagen and the GAG so as to gelatinize them or form a gel.

In the process of synthesizing a gel containing the collagen and the GAG, if the respective solutions of the collagen and the GAG are simply mixed together, a polyion complex (PIC) will be formed. In order to synthesize a gel homogeneously containing the collagen and the GAG, it is required to conduct the crosslinking reaction under a specific condition allowing the formation of PIC to be prevented.

A solution containing a phosphoric acid ion or a good solvent for collagen is used to prepare a buffer solution at pH value of 7.4, and glycosaminoglycan is mixed with the buffer solution. Under this condition, a homogeneous mixed solution of the collagen and the glycosaminoglycan can be obtained without forming any polyion complex.

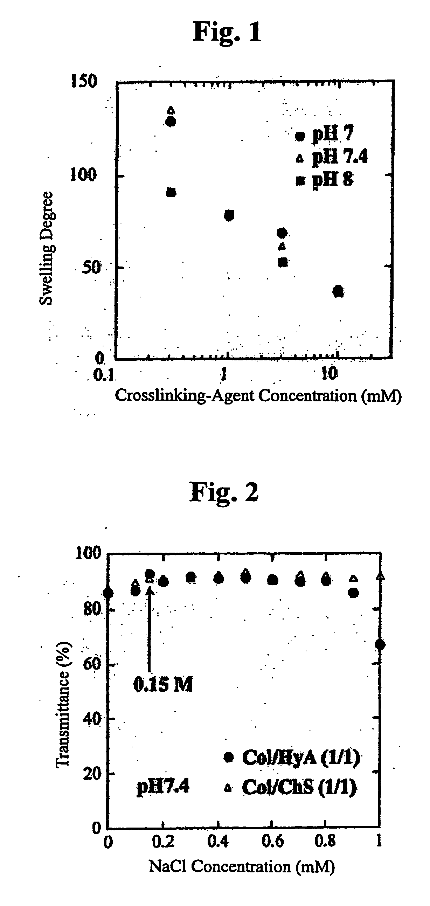

FIG 1 shows a swelling degree (= the weight of water in a gel /the dry weight of the gel) of type-II collagen crosslinked at various pH values with the above crosslinking agent. The swelling degree may be calculated by the formula [water content (%)] / [100 - water content (%)]. The swelling degree is reduced as the concentration of the crosslinking agent is increased. This means that the crosslinking is intensified as the concentration of the crosslinking agent is increased. However, if the crosslinking agent is added at 3 mM or more, the collagen will be undesirably precipitated to cause an inhomogeneous gel. Thus, the concentration of the crosslinking agent is preferably set in the range of 0.3 to 3 mM. As long as a homogeneous gel is obtained, the swelling degree is not limited to a specific value.

While the influence of the pH value is observed in the low concentration range (less than 0.3 mM) of the crosslinking agent, it is not observed in other range. The reaction time required for forming the gel is about 30 minutes when a reaction temperature is set at 4°C. By contrast, in the reaction temperature range of 25°C to 37°C, the gel formation is completed within 5 minutes. Thus, the reaction temperature for gel formation is preferably set in the range of 25°C to 37°C.

FIG 2 shows the test result of the transmittance in each product formed by crosslinking between collagen and hyaluronic acid (Col / HyA = 1 : 1) and between collagen and chondroitin sulfate (Col / ChS = 1 : 1) in pH 7.4 of phosphoric acid buffer solution added with a salt at various concentrations. It was verified that no PIC is formed under the condition of pH 7.4 irrespective of the salt concentration. This result means that the crosslinking reaction can be adequately conducted under physiological conditions, or can be adequately conducted in the presence of cells mixed with the solution in advance.

The above test was performed by measuring the transmittance of a light with a wavelength of 500 nm using a spectrophotometer. A transmittance of 100% means that the light fully transmits through the solution, or the solution is a homogeneous and transparent liquid. A transmittance of 0% means that the light cannot transmit therethrough at all, or some precipitate such as polyion complex is formed. If collagen and glycosaminoglycan are mixed together in water, the transmittance will be zero % due to the formation of a polyion complex. By contrast, when they are mixed together in a buffer solution containing a phosphoric acid ion, the transmittance of the mixed solution becomes approximately 100%, which shows that they are homogeneously mixed. The above data of transmittance verifies that a homogeneous mixed solution can be obtained at a physiological salt concentration as well as at a physiological pH value.

As with the case of collagen alone, a reaction system additionally including GAG can provide a gel in the range of 0.3 to 10 mM (0.1 to 10 mM in case of additionally including HyA), and the swelling degree is reduced as the concentration of the crosslinking agent to be added is increased. However, as with the case of collagen alone, if the crosslinking agent is added at 3 mM or more, the collagen will be undesirably precipitated to cause an inhomogeneous gel. In case of collagen plus GAG, the gel formation is also completed within 5 minutes when a reaction temperature is set at 37°C.

### [EXAMPLES]

### [Inventive Example 1]

A salt was added into 0.1 M phosphoric acid buffer solution of pH 7.4 (4°C) to establish physiological conditions (pH 7.4, 0.15M NaCl), and type-II collagen and 10 wt% of hyaluronic acid (HyA) are dissolved in the buffer solution. Then, a crosslinking agent was added at a concentration of 1.0 mM into the buffer solution. A pentaerythritol-based tetrafunctional polyethyleneglycol (unit number n = 56) having a succinimidyl group at the carboxyl terminal thereof was used as the crosslinking agent.

The mixed solution was sufficiently stirred, and then deaerated. Then, a crosslinking reaction was conducted in a hot water maintained at 37°C for 18 hours. As a result, a gel containing collagen and hyaluronic acid was synthesized. The obtained collagen-glycosaminoglycan complex matrix had a swelling degree (= the weight of water in the gel / the dry weight of the gel) of 108.8. No PIC was observed.

### [Inventive Example 2]

A synthesis was conducted under the same conditions as those in Inventive Example 1 except that the concentration of the crosslinking agent was set at 0.3 mM. The same gel as that in Inventive Example 1 was obtained. The obtained collagen-glycosaminoglycan complex matrix had a swelling degree of 177.6.

### [Inventive Example 3]

A synthesis was conducted under the same conditions as those in Inventive Example 1 except that the concentration of the crosslinking agent was set at 3 mM. The same gel as that in Inventive Example 1 was obtained. The obtained collagen-glycosaminoglycan complex matrix had a swelling degree of 83.8.

### [Inventive Example 4]

A synthesis was conducted under the same conditions as those in Inventive Example 1 except that chondroitin sulfate was used as a substitute for hyaluronic acid, and the concentration of the crosslinking agent was set at 1.0 mM. The same gel as that in Inventive Example 1 was obtained. The obtained collagen-glycosaminoglycan complex matrix had a swelling degree of 95.6.

### [Inventive Example 5]

A synthesis was conducted under the same conditions as those in Inventive Example 4 except that the concentration of the crosslinking agent was set at 0.3 mM. The same gel as that in Inventive Example 1 was obtained. The obtained collagen-glycosaminoglycan complex matrix had a swelling degree of 110.7.

### [Comparative Example 1]

A synthesis was conducted under the same conditions as those in Inventive Example 4 except that the concentration of the crosslinking agent was set at 0.1 mM. No gel was formed due to the excessively low concentration of the crosslinking agent.

### [Comparative Example 2]

A synthesis was conducted under the same conditions as those in Inventive Example 1 except that the concentration of the crosslinking agent was set at 10 mM. The obtained collagen-glycosaminoglycan complex matrix had a swelling degree of 22.1. The polyethyleneglycol chains provided the higher concentration of the crosslinking agent as compared to other Examples caused precipitation/sedimentation of the collagen, resulting in inhomogeneity in the obtained gel.

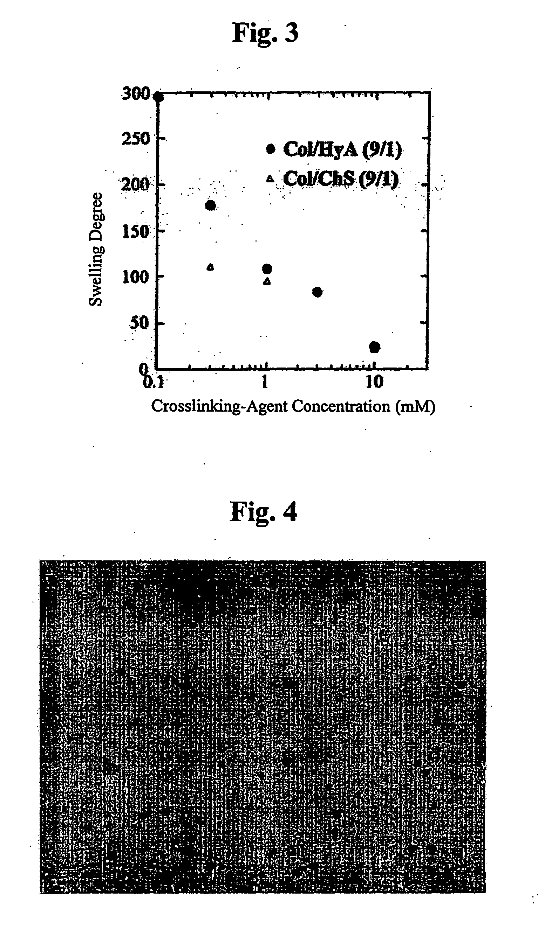

As seen in FIG. 3 showing the swelling degree of each of the collagen-glycosaminoglycan complex matrix obtained in Inventive Examples 1 to 5 and Comparative Examples 1 and 2, the swelling degree is reduced as the concentration of the crosslinking agent is increased.

### [Inventive Example 6]

A pentaerythritol-based tetrafunctional crosslinking agent was added into a buffer solution (pH 7.4, 0.15M NaCl) containing cartilage cells, collagen and glycosaminoglycan at a concentration of 1 × 10⁶ cells/mL, and the mixed solution was incubated at 37°C for 10 minutes. A photograph of the result is shown in FIG 4. All of circular spots in the photograph are the cartilage cells enclosed in an obtained gel. The cartilage cells are homogeneously dispersed over the collagen-hyaluronic acid gel, and can be obviously identified from a circular shape peculiar to a cartilage cell.

## Claims

1. A cartilage tissue regeneration matrix comprising of a biologically active glycosaminoglycan-polycation complex, which is formed by a homogeneous crosslinking reaction in the presence of cells using a polyfunctional crosslinking agent in a buffer solution under physiological conditions, or under the conditions of pH 7.0 to 8.0, 37°C and 0.1 to 0.2 M NaCl said crosslinking agent consisting of polyethyleneglycol having two or more electrophilic leaving groups at the carboxyl terminal thereof.

2. A method of preparing a cartilage tissue regeneration matrix comprising of a biologically active glycosaminoglycan-polycation complex, comprising mixing glycosaminoglycan and polycation in a buffer solution, and homogeneously crosslinking said glycosaminoglycan and polycation with a polyfunctional crosslinking agent under physiological conditions or under the conditions of pH 7.0, to 8.0 37°C and 0.1 to 0.2 M NaCl to synthesize said glycosaminoglycan-polycation complex, wherein said crosslinking agent consists of polyethyleneglycol having two or more electrophilic leaving groups at the carboxyl terminal thereof, and the concentration of said crosslinking agent is in the range of 0.3 to 3 mM.

3. The method as defined in claim 2, wherein the crosslinking reaction between said glycosaminoglycan and polycation is homogeneously conducted in the presence of cells mixed therewith in advance.

## Patentansprüche

1. Eine Matrix für die Regeneration von Knorpelgewebe, welche aus einem biologisch aktiven Glycosaminoglykan-Polykation-Komplex besteht, welcher durch eine homogene Vernetzungsreaktion in Gegenwart von Zellen gebildet wird, wobei ein polyfunktionelles Vernetzungsagens in einer Pufferlösung unter physiologischen Bedingungen, oder unter den Bedingungen von einem pH-Wert von 7,0 bis 8,0, 37°C und 0,1 bis 0,2 M NaCl, verwendet wird, wobei das Vernetzungsagens aus Polyethylenglykol mit zwei oder mehr elektrophilen Abgangsgruppen an seinem Carboxyl-Ende besteht.

2. Verfahren zur Vorbereitung einer Matrix für die Knorpelgeweberegeneration, welche aus einem biologisch aktiven Glycosaminoglykan-Polykation-Komplex besteht, das das Mischen von Glycosaminoglykan und einem Polykation in einer Pufferlösung umfasst und das homogene Vernetzen von Glycosaminoglykan und einem Polykation mit einem polyfunktionellen Vernetzungsagens unter physiologischen Bedingungen oder unter den Bedingungen von einem pH-Wert von 7,0 bis 8,0, 37°C und 0,1 bis 0,2 M NaCl, um den Glycosaminoglykan-Polykation-Komplex zu synthetisieren, wobei das Vernetzungsagens aus Polyethylenglykol mit zwei oder mehr elektrophilen Abgangsgruppen an seinem Carboxyl-Ende besteht, und wobei die Konzentration des Vernetzungsagens im Bereich von 0,3 bis 3 mM liegt.

3. Verfahren gemäß Anspruch 2, wobei die Vernetzungsreaktion zwischen dem Glycosaminoglykan und dem Polykation in Gegenwart von Zellen, die zuvor damit gemischt wurden, homogen durchgeführt wird.

## Revendications

1. Matrice de régénération de tissu de cartilage comprenant un complexe glycosaminoglycane-polycation biologiquement actif, qui est formé par une réaction de réticulation homogène en présence de cellules, utilisant un agent de réticulation polyfonctionnel dans une solution tampon dans des conditions physiologiques, ou dans les conditions de pH 7,0 à 8,0, 37°C et NaCl 0,1 M à 0,2 M, ledit agent réticulant étant constitué d'un polyéthylèneglycol ayant au moins deux groupes partants électrophiles à l'extrémité carboxyle.

2. Procédé de préparation d'une matrice de régénération de tissu de cartilage comprenant un complexe glycosaminoglycane-polycation biologiquement actif, comprenant le mélange d'un glycosaminoglycane et d'un polycation dans une solution tampon, et la réticulation homogène desdits glycosaminoglycane et polycation avec un agent réticulant polyfonctionnel dans des conditions physiologiques, ou dans les conditions de pH 7,0 à 8,0, 37°C et NaCl 0,1 M à 0,2 M, pour la synthèse dudit complexe glycosaminoglycane-polycation, dans lequel ledit agent réticulant est constitué d'un polyéthylèneglycol ayant au moins deux groupes électrophiles à l'extrémité carboxyle, et la concentration dudit agent réticulant se situe dans le domaine de 0,3 à 3 mM.

3. Procédé selon la revendication 2, dans lequel la réaction de réticulation entre ledit glycosaminoglycane et ledit polycation s'effectue de manière homogène en présence de cellules mélangées au préalable avec eux.
